# EUROPÄISCHE PATENTSCHRIFT

(11) **EP 1 567 209 B1**
(45) Veröffentlichungstag und Bekanntmachung des Hinweises auf die Patenterteilung: **21.01.2009**
(21) Anmeldenummer: 03811706.5
(22) Anmeldetag: 17.11.2003
(51) Int. Cl.: A61M 1/00

(54) **VORRICHTUNG ZUM VORRÜBERGEHENDEN FESTHALTEN EINER NADELSCHUTZKAPPE EINER INJEKTIONSVORRICHTUNG**
DEVICE FOR TEMPORARILY RETAINING A PROTECTIVE NEEDLE CAP OF AN INJECTION APPARATUS
DISPOSITIF DE MAINTIEN TEMPORAIRE D'UNE COIFFE DE PROTECTION D'AIGUILLE D'UN DISPOSITIF D'INJECTION

(30) Priorität: 25.11.2002 CH 198502
(43) Veröffentlichungstag der Anmeldung: 31.08.2005
(73) Patentinhaber: TecPharma Licensing AG, 3401 Burgdorf (CH)
(72) Erfinder: HOMMANN, Edgar, CH-3257 Grossaffoltern (CH)
(86) Internationale Anmeldenummer: PCT/CH2003/000756
(87) Internationale Veröffentlichungsnummer: WO 2004/047896

(56) Entgegenhaltungen:
- EP-A- 0 838 228
- WO-A-00/51667
- DE-U- 29 608 141
- FR-A- 2 714 836
- NL-C- 1 006 153
- US-A- 4 915 233
- US-A- 5 356 385

## Beschreibung

Die Erfindung betrifft eine Vorrichtung zum vorübergehenden Festhalten einer Nadelschutzkappe einer Injektionsvorrichtung, enthaltend ein Gehäuse, einen im Gehäuse angeordneten Aufnahmeteil zur Aufnahme der Injektionsvorrichtung und Haltemittel.

Bei üblichen, manuell zu betätigenden Injektionsspritzen wird in der Regel die Nadelschutzkappe vor dem Gebrauch von Hand abgenommen und nach dem Gebrauch von Hand wieder aufgesetzt. Gerade beim zuletzt genannten Vorgang besteht für den Benutzer eine erhebliche Verletzungsgefahr durch die gebrauchte Injektionsnadel. Es wurden deshalb schon verschiedene Hilfsmittel zum Abziehen und insbesondere zum Aufsetzen von Nadelschutzkappen vorgeschlagen, die aber allesamt einen separaten Arbeitsschritt erfordern.

Aus dem Dokument US 5,356,385 ist ein Halter für Nadelkappen bekannt, bei dem eine Injektionsspritze durch eine Öffnung in ein Haltergehäuse und in dem Gehäuse in einen Greifmechanismus mit einer Drehplatte eingeführt werden kann. Der Greifinechanismus umfasst diverse Klemmelemente, die an der Drehplatte schwenkbar angekoppelt sind. Eine Drehung der Drehplatte bewirkt ein Verschwenken der Klemmelemente. Die Drehplatte ist mittels einer Feder derart vorgespannt, dass sich die Klemmelemente in einer Halteposition zum Halten der Nadelschutzkappe befinden. Um eine Spritze in den Halter einführen zu können, muss vorher manuell ein Arm (60) eingedrückt werden, der die Drehung der Drehplatte derart bewirkt, dass die Klemmelemente von ihrer Halteposition in eine Freigabeposition geschwenkt werden. Beim Lösen des Armes wird die Drehplatte mittels ihrer Vorspannung zurück in die Halteposition gedreht, sodass die Klemmelemente an der Nadelschutzkappe angreifen. Wird nun die Spritze aus dem Halter gezogen, verbleibt die Nadelschutzkappe im Halter, da sie von den Klemmelementen festgehalten wird.

Die Erfindung hat die Aufgabe, eine Vorrichtung vorzuschlagen, mit welcher die Nadelschutzkappe bei einem ohnehin erforderlichen Arbeitsgang zur Vorbereitung einer Injektionsvorrichtung abgezogen und danach auch wieder aufgesetzt werden kann.

Zur Lösung dieser Aufgabe ist die erfindungsgemässe Vorrichtung dadurch gekennzeichnet, dass der Aufnahmeteil im Gehäuse bewegbar ist, dass in einer ersten Lage des Aufnahmeteils die Haltemittel in einer Freigabeposition sind und dass in einer zweiten Lage des Aufnahmeteils die Haltemittel in einer Halteposition sind.

Nach einer Ausführungsart der Erfindung ist der Aufnahmeteil durch eine Drehung von seiner ersten Lage in seine zweite Lage bewegbar. Diese Lösung ist insbesondere für Injektionsvorrichtungen vorteilhaft, die zwecks Einsetzens und Entnehmens einer Spritze drehend auseinander genommen werden. Die Haltemittel werden gleichzeitig mit diesem Drehen betätigt.

Gemäss einer weiteren Ausführungsart der Erfindung sind die Haltemittel als Klemmstücke ausgebildet, die um parallel zur Längsachse der Vorrichtung verlaufende Schwenkachsen gegen die Längsachse hin schwenkbar sind. Dies ermöglicht einen besonders einfachen Aufbau der Vorrichtung. Wenn nach weiteren Ausführungsarten der Erfindung der Aufnahmeteil eine Andrückkurve und gegebenenfalls eine Lösekurve enthält, ergibt sich eine Zwangssteuerung der Klemmteile, was einen besonders sicheren Betrieb der Vorrichtung ermöglicht.

Nach einer weiteren Ausführungsart der Erfindung sind Anzeigemittel vorgesehen, welche von aussen sichtbar machen, in welcher Drehlage der Aufnahmeteil sich befindet. Die Anzeigemittel enthalten vorzugsweise ein Fenster im Gehäuse, durch welches in jeder Drehlage des Aufnahmeteils ein anderer, an diesem vorhandener Markierbereich sichtbar ist. Diese Massnahmen machen den Betrieb der Vorrichtung noch bequemer und sicherer.

Ein anderer Aspekt der Erfindung betrifft eine Ladestation für die Vorbereitung einer Injektionsvorrichtung, welche eine erfindungsgemässe Vorrichtung enthält. Dabei kann die Ladestationen noch andere funktionelle Ele- , mente enthalten, die der Vorbereitung einer Injektionsvorrichtung, insbesondere eines Autoinjektors, dienen. Die erfindungsgemässe Vorrichtung ist vorzugsweise herausnehmbar in der Ladestation aufgenommen. Eine besondere Ausführungsart der Ladestation ist gleichzeitig als Behältnis zur Aufbewahrung der Vorrichtung und von Teilen einer Injektionsvorrichtung ausgebildet. Dies ermöglicht dem Benutzer einer Injektionsvorrichtung, die benötigten Einzelteile einfach mit sich zu tragen und übersichtlich aufzubewahren.

Eine Ausführungsart der Erfindung wird nachfolgend unter Bezugnahme auf die beiliegenden Zeichnungen beispielhaft beschrieben. Es zeigt:
- Figur 1: eine aufgeschnittene, perspektivische Ansicht des Vorderteils eines Autoinjektors,
- Figur 2: eine Explosionsansicht eines Ausführungsbeispiels der Erfindung,
- Figur 3: eine perspektivische Ansicht einer Einzelheit aus Figur 2,
- Figur 4: einen Querschnitt durch den unteren Teil der Vorrichtung nach Figur 2 und
- Figur 5: eine Ladestation mit einer Vorrichtung nach Figur 2.

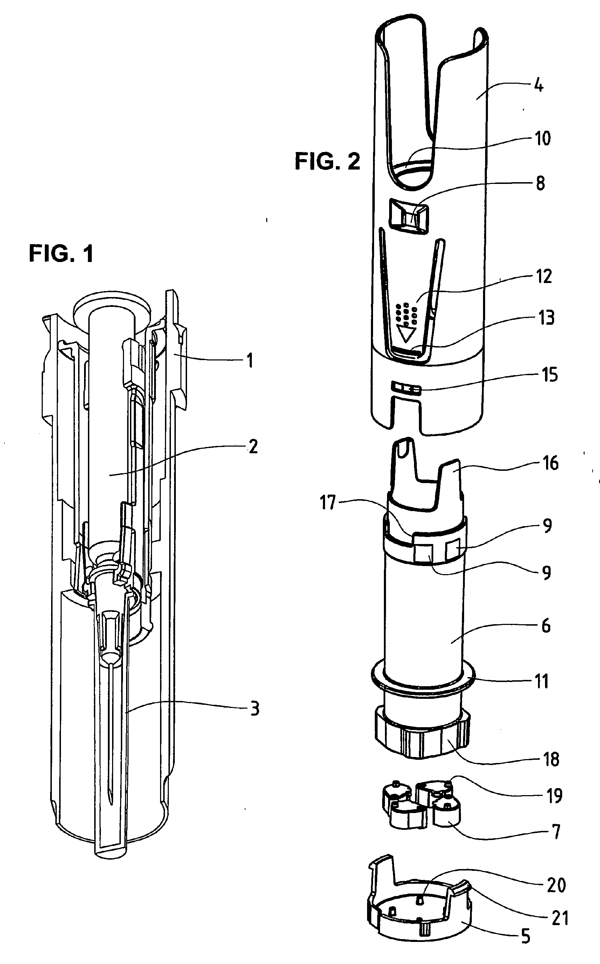

In Figur 1 ist zum Besseren Verständnis des Zusammenhangs der Vorderteil 1 eines Autoinjektors mit darin aufgenommener Spritze 2 dargestellt. Der Vorderteil 1 wird vor der Verwendung des Autoinjektors mittels einer bajonettartigen Verbindung mit einem nicht dargestellten Antriebsteil zusammengesetzt, der die zur automatischen Ausführung einer Injektion erforderlichen Elemente, insbesondere Federn enthält. Die Injektionsnadel dieser Spritze 2 ist in bekannter Weise mit einer aufgesteckten Nadelschutzkappe 3 abgedeckt. Die erfindungsgemässe Vorrichtung dient dazu, vor der Verwendung des Autoinjektors diese Nadelschutzkappe 3 abzuziehen und sie nach der Verwendung des Autoinjektors wieder aufzusetzen. Dazu nutzt die Erfindung die Vorgänge des Zusammensetzens und Auseinandernehmens des Autoinjektors vor beziehungsweise nach der Injektion.

Die in den Figuren 2 bis 5 dargestellte Vorrichtung 30 besteht aus einem Gehäuse 4, das unten mit einem Gehäuseboden 5 verschlossen ist, welcher durch Rastnocken 21 gehalten ist, die in Öffnung 15 des Gehäuses 4 eingreifen. Im Gehäuse 4 ist eine Kurvenhülse 6 drehbar gehalten, wobei die Drehbarkeit durch an der Kurvenhülse 6 angeordnete Anschläge 17 begrenzt wird, die mit einer in den Figuren nicht sichtbaren Rippe im Gehäuse 4 zusammenwirken. Ein im Gehäuse 4 angeordnetes Fenster 8 gibt entsprechend der jeweiligen Drehlage der Kurvenhülse 6 den Blick auf einen der beiden an ihr vorgesehenen, vorzugsweise farbigen Markierbereiche 9 frei. Ein im oberen Bereich des Gehäuses 4 angeordneter Innenflansch 10 stützt die Kurvenhülse 6 sowohl radial als auch axial und im unteren Bereich der Kurvenhülse 6 ist ein Aussenflansch 11 vorgesehen, mit dem die Kurvenhülse 6 radial im Gehäuse 4 geführt wird. Die Kurvenhülse hat die Aufgabe, in ihrem Inneren den Vorderteil 1 eines Autoinjektors aufzunehmen. Nach oben ragende Lappen 16 und/oder im Inneren der Kurvenhülse vorgesehene, nicht dargestellte Rippen sorgen für eine drehfeste Verbindung zwischen der Kurvenhülse 6 und dem Vorderteil 1 des Autoinjektors.

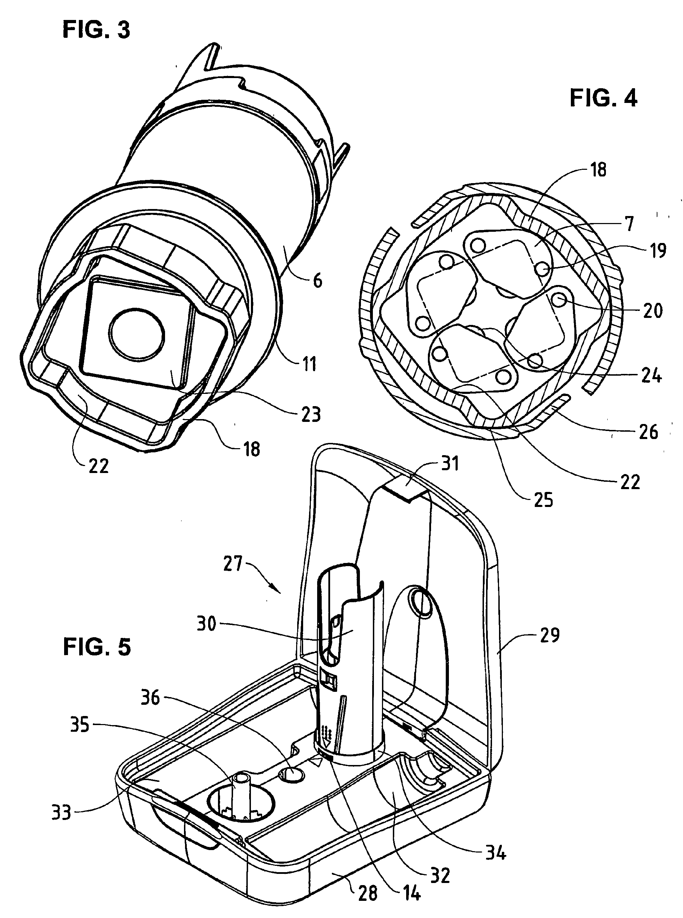

Vom Gehäuseboden 5 stehen vier Lagerzapfen 20 nach oben vor, auf denen vier Klemmstücke 7 schwenkbar gelagert sind. Diese Klemmstücke 7 haben die Aufgabe, die Nadelschutzkappe 3 zu ergreifen und festzuhalten, wenn der Autoinjektor zusammengesetzt wird und umgekehrt die Nadelschutzkappe 3 wieder freizugeben, wenn der Autoinjektor zwecks Entnahme der verbrauchten Spritze 2 wieder auseinander genommen wird. Wie Figur 4 zeigt, sind an den Klemmstücken 7 Greifrippen 24 vorgesehen, welche in das relativ weiche Material der Nadelschutzkappe 3 eingedrückt werden, um diese sicher zu greifen. Die Bewegung der Klemmstücke 7 wird wie folgt durch einen unten an der Kurvenhülse 6 geformten Kurventeil 18 gesteuert. Im Inneren des Kurventeils befindet sich eine Andrückkurve 22, welche die Klemmstücke 7 nach innen drückt, wenn die Kurvenhülse 6 im Uhrzeigersinn gedreht wird. Dies ist am Besten in Figur 4 zu erkennen. Wird die Kurvenhülse 6 im Gegenuhrzeigersinn gedreht, greift eine innerhalb der Andrückkurve 22 angeordnete Lösekurve 23, die am Besten in Figur 3 zu erkennen ist, an Steuerzapfen 19 an, die auf jedem Klemmstück 7 nach oben vorstehend angeordnet sind und drängt die Klemmstücke 7 nach außen. Der Kurventeil 18 der Kurvenhülse 6 ist mit seinem Aussenumfang 25 im Gehäuseboden 5 geführt, wobei am Gehäuseboden 5 angeformte Zungen 26 mit dem Aussenbereich 25 derart zusammenwirken, dass die Kurvenhülse 6 in ihren beiden Dreh-Endlagen einrastet.

Zur Verwendung der Vorrichtung wird diese entweder mit einer Hand festgehalten oder vorteilhafter in eine entsprechende Ladestation 27 eingesetzt, wie dies weiter unten beschrieben wird. Dann wird von oben der Vorderteil 1 eines Autoinjektors in die Kurvenhülse 6 eingesetzt und in den Vorderteil 1 wird eine mit einem Wirkstoff gefüllte Spritze mit Injektionsnadel und Nadelschutzkappe 3 gesteckt. Dann wird der Antriebsteil des Autoinjektors aufgesetzt und im Uhrzeigersinn gedreht. Bei dieser Drehung wird - gleichzeitig oder nacheinander, je nach der zwischen den einzelnen Komponenten wirkenden Reibung - der Antriebsteil des Autoinjektors mit dem Vorderteil 1 über die erwähnte bajonettartige Verbindung gekoppelt und die Kurvenhülse 6 im Gehäuse 4 gedreht, wobei die Klemmstücke 7 durch die Andrückkurve 22 nach innen gedrängt werden und die Nadelschutzkappe 3 zwischen sich festklemmen. Im Fenster 8 ist nun ein anderer, beispielsweise grün gefärbter Bereich 9 sichtbar, der signalisiert, dass der Autoinjektor nun betriebsbereit ist und nach oben herausgezogen werden kann, wobei die Nadelschutzkappe 3 zwischen den Klemmstücken 7 verbleibt. Nach der Injektion spielt sich das Ganze in umgekehrter Reihenfolge ab, der Autoinjektor wird mit seinem Vorderteil 1 in die Kurvenhülse 6 gesteckt, die Injektionsnadel schiebt sich in die Nadelschutzkappe 3 und der Antriebsteil des Autoinjektors wird im Gegenuhrzeigersinn gedreht, um seine Verbindung mit dem Vorderteil 1 zu lösen. Die Kurvenhülse wird durch diese Drehung mitgenommen und die Lösekurve 23 sorgt dafür, dass die Klemmstücke 7 die Nadelschutzkappe freigeben. Schliesslich kann die Spritze 2 mitsamt der wieder aufgesetzten Nadelschutzkappe 3 aus dem Vorderteil 1 entnommen werden.

Figur 5 zeigt eine Ladestation 27 für einen Autoinjektor, in welcher die oben beschriebene Vorrichtung 30 in Gebrauchslage gehalten ist. Im Gehäuse 4 der Vorrichtung sind Federzungen 12 mit Rastnasen 13 vorhanden, welche in Rast-Ausnehmungen 14 einrasten, die in einer Aufnahme 34 der Ladestation 27 vorgesehen sind. Die Ladestation ist im dargestellten Beispiel gleichzeitig ein Behältnis, bestehend aus einem Unterteil 28, einem Deckel 29 und einem Verschluss 31. Neben der beschriebenen Aufnahme 34 für die Vorrichtung 30 enthält das dargestellte Beispiel eine Aufnahmemulde 32 für den Antriebsteil des Autoinjektors, eine Aufnahmemulde 33 für die Vorrichtung 30, einen Spannzapfen 35 zum Spannen der Federn des Autoinjektors und eine weitere Aufnahme 36 für ein Werkzeug wie einen Schlüssel zum Anziehen und Lösen einer Injektionsnadel des Typs Luer-Lock.

## Patentansprüche

1. Vorrichtung zum vorübergehenden Festhalten einer Nadelschutzkappe (3) einer Injektionsnadel einer Injektionsvorrichtung, enthaltend ein Gehäuse (4), einen im Gehäuse (4) angeordneten Aufnahmeteil (6) zur Aufnahme der Injektionsvorrichtung und Haltemittel (7), **dadurch gekennzeichnet, dass** der Aufnahmeteil (6) im Gehäuse (4) durch Drehung der darin aufgenommenen Injektionsvorrichtung bewegbar ist, dass in einer ersten Lage des Aufnahmeteils (6) die Haltemittel (7) in einer Freigabeposition sind und dass in einer zweiten Lage des Aufnahmeteils (6) die Haltemittel (7) in einer Halteposition sind.

2. Vorrichtung nach Anspruch 1, **dadurch gekennzeichnet, dass** die Drehung des Aufnahmeteils (6) eine Zwangssteuerung der Haltemittel (7) bildet.

3. Vorrichtung nach Anspruch 1 oder 2, **dadurch gekennzeichnet, dass** die Haltemittel als Klemmstücke (7) ausgebildet sind, die um parallel zur Längsachse der Vorrichtung verlaufende Schwenkachsen (20) gegen die Längsachse hin schwenkbar sind.

4. Vorrichtung nach Anspruch 3, **dadurch gekennzeichnet, dass** der Aufnahmeteil (6) eine Andrückkurve (22) enthalt, welche in der zweiten Drehlage des Aufnahmeteils (6) die Klemmstücke (7) zur Längsachse der Vorrichtung hin drückt

5. Vorrichtung nach Anspruch 3 oder 4, **dadurch gekennzeichnet, dass** der Aufhahmetell (6) eine Lösekurve (23) enthalt, welche in der ersten Drehlage des Aufnahmeteils (6) die Klemmstücke (7) von der Längsachse der Vorrichtung weg drückt.

6. Vorrichtung nach einem der vorangehenden Ansprüche, **gekennzeichnet durch** Anzeigemittel (8), welche von aussen sichtbar machen, in welcher Drehlage der Aufnahmeteil (6) sich befindet.

7. Vorrichtung nach Anspruch 6, **dadurch gekennzeichnet, dass** die Anzeigemittel ein Fenster (8) im Gehäuse (4) enthalten, durch welches in jeder Drehlage des Aufnahmeteils (6) ein anderer, an diesem vorhandener Markierbereich (9) sichtbar ist.

8. Ladestation (27) für die Vorbereitung einer Injektionsvorrichtung, **dadurch gekennzeichnet, dass** sie eine Vorrichtung (30) nach einem der vorangehenden Ansprüche enthält.

9. Ladestation nach Anspruch 8, **dadurch gekennzeichnet, dass** die Vorrichtung (30) herausnehmbar in der Ladestation (27) aufgenommen ist.

10. Ladestation nach Anspruch 8 oder 9, **dadurch gekennzeichnet, dass** sie als Behältnis (28, 29) zur Aufbewahrung von Teilen einer Injektionsvorrichtung ausgebildet ist.

11. Ladestation nach einem der Ansprüche 8 bis 10, **dadurch gekennzeichnet, dass** der Aufnahmeteil (6) zum Einsetzen eines Vorderteils (1) eines Autoinjektors ausgebildet ist, ein Antriebsteil des Autoinjektors mit dem Vorderteil (1) koppelbar ist, wobei durch Drehung des Antriebsteils die Kopplung und die Drehung des Aufnahmeteils (6) vermittelbar ist.

## Claims

1. A device for temporarily retaining a needle protection cap (3) of an injection needle of an injection apparatus, comprising a housing (4), a receiving portion (6) arranged in the housing (4) for receiving the injection apparatus, and holding means (7), **characterised in that** the receiving portion (6) is movable in the housing (4) by rotation of the injection apparatus received therein, that the holding means (7) are in a release position in a first position of the receiving portion (6) and the holding means (7) are in a holding position in a second position of the receiving portion (6).

2. A device according to claim 1 **characterised in that** the rotation of the receiving portion (6) forms a positive control of the holding means (7).

3. A device according to claim 1 or claim 2 **characterised in that** the holding means are in the form of clamping portions (7) which are pivotable towards the longitudinal axis about pivot axes (20) extending parallel to the longitudinal axis of the device.

4. A device according to claim 3 **characterised in that** the receiving portion (6) includes a pressure cam (22) which in the second rotational position of the receiving portion (6) urges the clamping portions (7) towards the longitudinal axis of the device.

5. A device according to claim 3 or claim 4 **characterised in that** the receiving portion (6) includes a release cam (23) which in the first rotational position of the receiving portion (6) urges the clamping portions (7) away from the longitudinal axis of the device.

6. A device according to one of the preceding claims **characterised by** display means (8) which make the rotational position in which the receiving portion (6) is disposed visible from the exterior.

7. A device according to claim 6 **characterised in that** the display means include a window (8) in the housing (4), through which it is possible to see in each rotational position of the receiving portion (6) another marking region (9) present thereon.

8. A loading station (27) for the preparation of an injection apparatus **characterised in that** it includes a device (30) according to one of the preceding claims.

9. A loading station according to claim 8 **characterised in that** the device (30) is removably received in the loading station (27).

10. A loading station according to claim 8 or claim 9 **characterised in that** it is in the form of a container (28, 29) for storing parts of an injection apparatus.

11. A loading station according to one of claims 8 to 10 **characterised in that** the receiving portion (6) is adapted for the insertion of a front portion (1) of an autoinjector, and a drive portion of the autoinjector can be coupled to the front portion (1), wherein coupling and rotation of the receiving portion (6) can be implemented by rotation of the drive portion.

## Revendications

1. Dispositif de maintien temporaire d'un capuchon de protection (3) d'une aiguille d'injection d'un dispositif d'injection, contenant une partie formant logement (4), une partie réceptrice (6) disposée dans le logement (4) destinée à recevoir le dispositif d'injection et le dispositif d'arrêt (7), **caractérisé en ce que** la partie réceptrice (6) est mobile dans le logement (4) par la rotation du dispositif d'injection logé à l'intérieur, **en ce que**, dans une première position de la partie réceptrice (6), les dispositifs d'arrêt (7) sont dans une position débloquée et **en ce que**, dans une deuxième position de la partie réceptrice (6), les dispositifs d'arrêt (7) sont dans une position d'arrêt.

2. Dispositif selon la revendication 1, **caractérisé en ce que** la rotation de la partie réceptrice (6) forme une commande forcée des dispositifs d'arrêt (7).

3. Dispositif selon la revendication 1 ou 2, **caractérisé en ce que** les dispositifs d'arrêt sont conçus comme des pièces de serrage (7) qui peuvent être pivotées autour de l'axe de pivotement (20) parallèle à l'axe longitudinal du dispositif, contre l'axe longitudinal.

4. Dispositif selon la revendication 3, **caractérisé en ce que** la partie réceptrice (6) contient une courbe de pression (22) qui, dans la deuxième position de rotation de la partie réceptrice (6), appuie les pièces de serrage (7) sur l'axe longitudinal du dispositif.

5. Dispositif selon la revendication 3 ou 4, **caractérisé en ce que** la partie réceptrice (6) contient une courbe de desserrage (23) qui, dans la première position de rotation de la partie réceptrice (6), relâche la pression des pièces de serrage (7) sur l'axe longitudinal du dispositif.

6. Dispositif selon l'une des revendications précédentes, **caractérisé par** des dispositifs d'affichage (8) qui permettent de voir de l'extérieur, dans quelle position de rotation se trouve la partie réceptrice (6).

7. Dispositif selon la revendication 6, **caractérisé en ce que** les dispositifs d'affichage incluent une fenêtre (8) dans la partie formant logement (4), à travers laquelle il est possible de voir dans chaque position de rotation de la partie réceptrice (6) un secteur de repère existant (9) sur cette partie réceptrice.

8. Poste de charge (27) pour la préparation d'un dispositif d'injection, **caractérisé en ce qu'**il contient un dispositif (30) selon l'une des revendications précédentes.

9. Poste de charge selon la revendication 8, **caractérisé en ce que** le dispositif (30) est logé de façon détachable dans le poste de charge (27).

10. Poste de charge selon la revendication 8 ou 9, **caractérisé en ce qu'**il est conçu comme un récipient (28, 29) pour la conservation des pièces d'un dispositif d'injection.

11. Poste de charge selon l'une des revendications 8 à 10, **caractérisé en ce que** la partie réceptrice (6) est conçue pour loger une partie avant (1) d'un injecteur automatique, **en ce qu'**un élément d'entraînement de l'injecteur automatique peut être couplé avec la partie avant (1), la rotation de l'élément d'entraînement permettant de procurer le couplage et la rotation de la partie réceptrice (6).
